# EUROPEAN PATENT APPLICATION

(11) **EP 4 623 926 A1**
(43) Date of publication of application: **01.10.2025**
(21) Application number: 24166155.2
(22) Date of filing: 26.03.2024
(51) Int. Cl.: A61K 38/18, A61K 9/00, A61P 25/06

(54) **INTRANASAL ADMINISTRATION OF BDNF FOR TREATMENT OF PRIMARY HEADACHES**

(71) Applicant: Dompe' Farmaceutici SpA, 20122 Milano (IT)
(72) Inventor: GRECO, Rosaria, 27100 Pavia (IT); TASSORELLI, Cristina, 27100 Pavia (IT); DE FILLIPIS, Lidia, 20122 Milano (IT); ARAMINI, Andrea, 67100 L'Aquila (IT)
(74) Representative: Mauri, Elisabetta Maria Ester

(57) **Abstract**

The present invention relates to brain derived nerve factor (BDNF) for use in the acute or preventive treatment of cluster headache or migraine attacks in a subject, wherein said BDNF is administered intranasally to said subject.

## Description

### FIELD OF THE INVENTION

The present invention relates to the acute or preventive treatment of primary headaches, in particular migraine and cluster headache.

### STATE OF THE ART

According to the third edition of the International Classification of Headache Disorders published in 2018, headaches can be divided into primary headaches, secondary headaches, neuropathies or facial pains, and other headaches.

Primary headache disorders, which include migraine and cluster headache (CH), are the leading causes of disability worldwide and are caused by independent patho-mechanisms, differently from secondary headaches that develop as secondary symptoms to other disorders or infections. Owing to their unclear cause and the severe and prolonged pain, primary headache disorders often become refractory.

The headache phenotype varies among these primary headaches. In particular, while the pain associated with migraine is generally localized in the frontal, temporal, parietal, occipital and upper cervical regions, the pain in cluster headache is predominantly peri-orbital, in and around the eye, or temporal.

Notwithstanding these differences, both are thought to be mediated by the activation of the trigeminovascular system and are commonly considered as neurovascular disorders.

A rich plexus of nociceptive nerve fibers (non-myelinated C-fibers and thinly myelinated Aδ-fibers) originating in the trigeminal ganglion (TG) innervates vascular intracranial structures, including the pial, arachnoid and dural blood vessels as well as large cerebral arteries.

The axonal terminals of these collective nociceptive nerve fibers contain vasoactive neuropeptides, including calcitonin gene-related peptide (CGRP), substance P (SP), neurokinin A (NKA), vasoactive intestinal peptide (VIP), and pituitary adenylate cyclase-activating peptide-38 (PACAP38), that may be released upon stimulation of the trigeminal nerves, causing vasodilation of dural and pial vessels, and a potential cascade of events that results in activation and sensitization of the trigeminovascular system.

High concentrations of VIP, PACAP38, CGRP, have been reported in the serum of patients affected by migraine or CH (see for example Kuburas, A. et al, J Headache Pain 2023, v. 24, p. 34; Tanaka, M. et al., Cells 2023, 12, 2649; Pellesi L at al., Front. Neurol. 2022, 13: 871176; Edvinsson, L. et al., J Headache Pain 2018, 19, 21; Riesco N. et al., Curr Pain Headache Rep. 2017, 21(4):18; Pellesi L et al., Cephalalgia. 2022, 42(8): 687-695). Also, an increase of pro-inflammatory cytokines such as IL1β, as well as the decrease of anti-inflammatory cytokines such as IL-10, have been observed in the serum of patients affected by migraine or CH (see for example, Fidan I. et al, J Neuroimmunology 2006, 171:184-188; Geng C. et al., J Clin Neurosci 2022, 98:213-218; Oliveira AB, et al., J Neuroimmunology 2017, 313: 138-144; Chaudhry SR et al., Brain Stimul. 2019, 12(3): 643-651; Thuraiaiyah J et al., Cephalalgia. 2022, 42(14):1565-1588; Guo Z, et al, Neurobiol Pain 2022, 13;12:100096; Uzar E, et al, Eur Rev Med Pharmacol Sci. 2011, 15(10): 1111-6).

Currently, the main strategies for the treatment of both CH and migraine can be classified into two categories: acute treatments that aim to interrupt the single attacks, and preventive treatments that aim to reduce attack frequency, duration and intensity. Most patients are treated simultaneously with acute and preventive treatments.

As regards acute pharmacological treatments, the most used drug during CH and migraine attacks is subcutaneous sumatriptan, a serotonin receptor agonist for 5-HT1B and 5-HT1D, which provides rapid pain relief in most subjects within 20 minutes of injection. This drug has been shown to be safe in healthy patients but presents contraindications in case of concomitant cardiovascular, metabolic and cerebrovascular disorders (Hoskin KL, Goadsby PJ, Exp Neurol 1998, 150:45-51 6; Humphrey PP et al, Cephalalgia 1994, 14:401-410; Nature Reviews, 2018, doi: 10.1038/nrdp.2018.6).

Available preventive treatments consist of the administration of Verapamil, corticosteroids or lithium; however, these therapies have limited efficacy or display side effects over long-term treatment (Nature Reviews, 2018, doi: 10.1038/nrdp.2018.6).

Therefore, there is still a great need for novel and more effective therapies for cluster headache and migraine, thus overcoming the limitations of known current therapeutic strategies.

Brain-derived neurotrophic factor (BDNF) is one of the most important growth factors in the CNS (Barde YA et al. Brain derived neurotrophic factor. Prog Brain Res 1987; 71: 185-189.) and it plays a vital role in the development of CNS and neuronal plasticity. BDNF is involved in diverse brain diseases (Autry AE et al., Brain derived neurotrophic factor and neuropsychiatric disorders. Pharmacol Rev 2012; 64: 238-258) including schizophrenia, intellectual disability and autism, as well as depression and other affective disorders (Bjorkholm C, Monteggia LM. BDNF - a key transducer of antidepressant effects. Neuropharmacology 2016; 102: 72-79.). It is also a crucial neuromodulator in pain transmission.

In a bi-center prospective study, both migraine and CH patients revealed significantly higher BDNF serum levels during migraine attacks compared with headache-free periods, patients with tension-type headache, and healthy controls (Fischer, M., Wille, G., Klien, S. et al. Brain-derived neurotrophic factor in primary headaches. J Headache Pain 13, 469-475 (2012)).

### SUMMARY OF THE INVENTION

The present inventors have now surprisingly found that intranasally-administered BDNF is effective in mitigating trigeminal hyperalgesia in a model of primary headache associated with activation of the trigeminovascular system, based on nitroglycerin (NTG) administration, which is representative of cluster headache (CH) and migraine.

In particular, in the NTG-induced rat model, intranasally-administered BDNF dampens the enhancement of face rubbing behaviour upon formalin stimulus mirroring the hyperalgesia typical of subjects suffering from cluster headache or migraine, and it is also effective in decreasing the levels of vasoactive neuropeptides and inflammatory mediators involved in the pathophysiology of migraine and cluster headache.

In particular, it induces a marked reduction of VIP, PACAP38 and CGRP, a marked reduction of IL1β, as well as a marked increase of IL-10.

The above results support the role of BDNF in both acute and preventive treatment of CH or migraine attacks.

Accordingly, a first object of the invention is BDNF for use in the acute or preventive treatment of migraine or cluster headache attacks in a subject, wherein said BDNF is administered intranasally to said subject.

A further object of the invention is a pharmaceutical composition comprising BDNF, for use in the acute or preventive treatment of migraine or cluster headache attacks in a subject, said composition being administered intranasally to said subject.

### BRIEF DESCRIPTION OF THE FIGURES

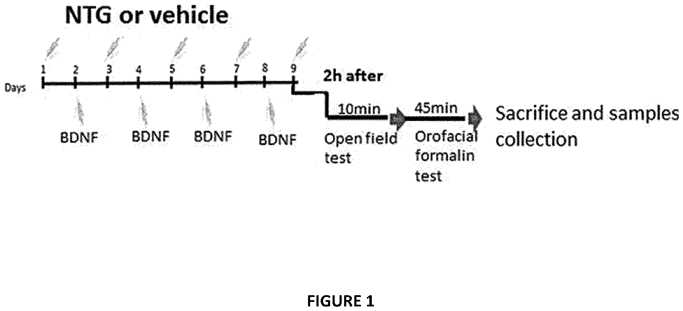

**Figure 1** shows a schematic representation of the experimental design of Example 1.

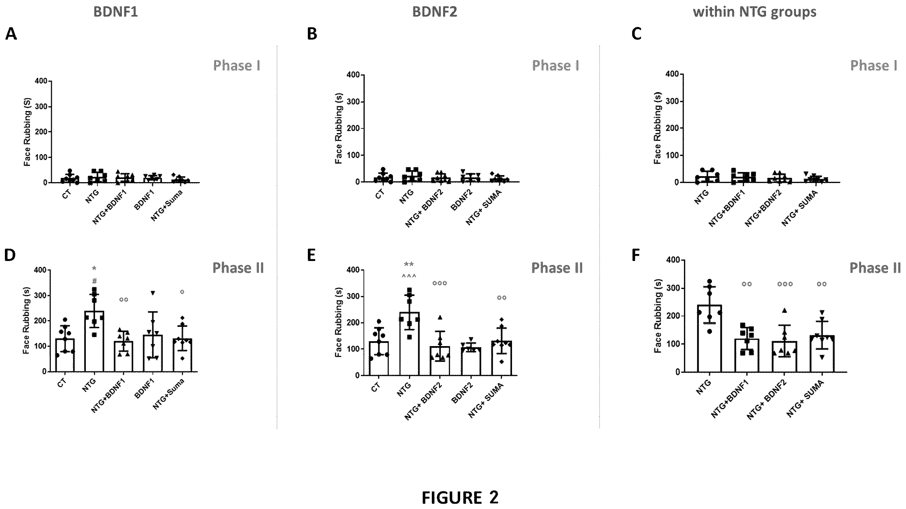

**Figure 2** shows the time spent (expressed in seconds) in rubbing, during 0-3 min (Phase I) and 12-45 min (Phase II) after formalin injection, by "CT", "NTG", "NTG+BDNF1", "BDNF1" and "NTG+SUMA" rat groups (panels A and D), "CT", "NTG", "NTG+BDNF2", "BDNF2" and "NTG+SUMA" rat groups (panels B and E), and "NTG", "NTG+BDNF1", "NTG+BDNF2", and "NTG+SUMA" rat groups (panels C and F), as described in Example 1. Data were tested for normality using Shapiro-Wilk test and considered normal. One-way ANOVA followed by Turkey's multiple comparisons test; *p<0.05 and **p<0.01 vs CT; °p<0.05, °°p<0.01 and °°°p<0.001 vs NTG; #p<0.05 vs BDNF1 ; ^^^p<0.001 vs BDNF2.

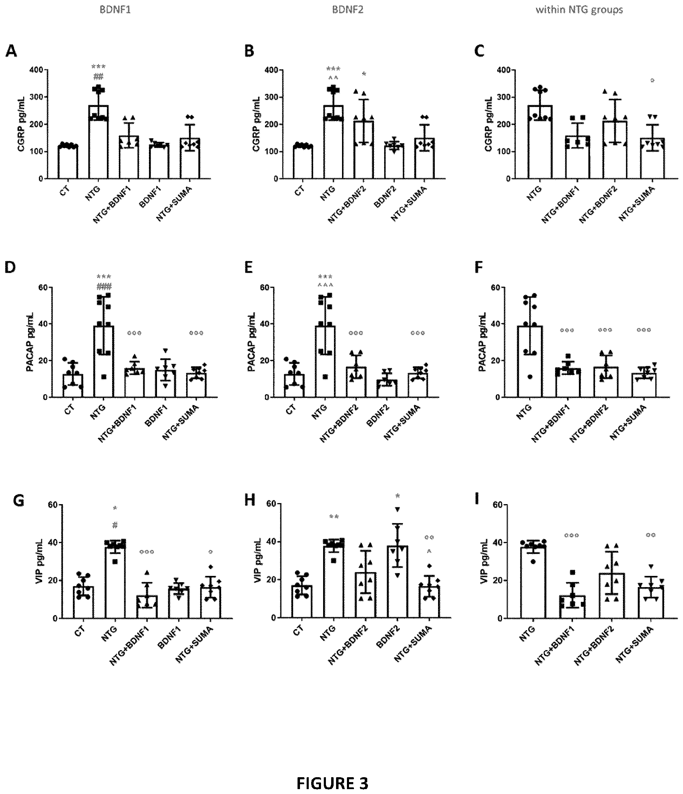

**Figure 3** shows the analysis in "CT", "NTG", "NTG+BDNF1", "BDNF1" and "NTG+SUMA" rat groups of serum concentration (pg/ml) of CGRP (panel A), PACAP38 (panel D), and VIP (panel G); the analysis in "CT", "NTG", "NTG+BDNF2", "BDNF2" and "NTG+SUMA" rat groups of serum concentration (pg/ml) of CGRP (panel B), PACAP38 (panel E), and VIP (panel H), and the analysis in "NTG", "NTG+BDNF1", "NTG+BDNF2", and "NTG+SUMA" rat groups of serum concentration (pg/ml) of CGRP (panel C), PACAP38 (panel F), and VIP (panel I). Kruskal-Wallis's test followed by Dunn's post-hoc test; *p<0.05 and **p<0.01 vs CT; °p<0.05, °°p<0.01 and °°°p<0.001 vs NTG; #p<0.05 vs BDNF1 ; ^p<0.05 vs BDNF2.

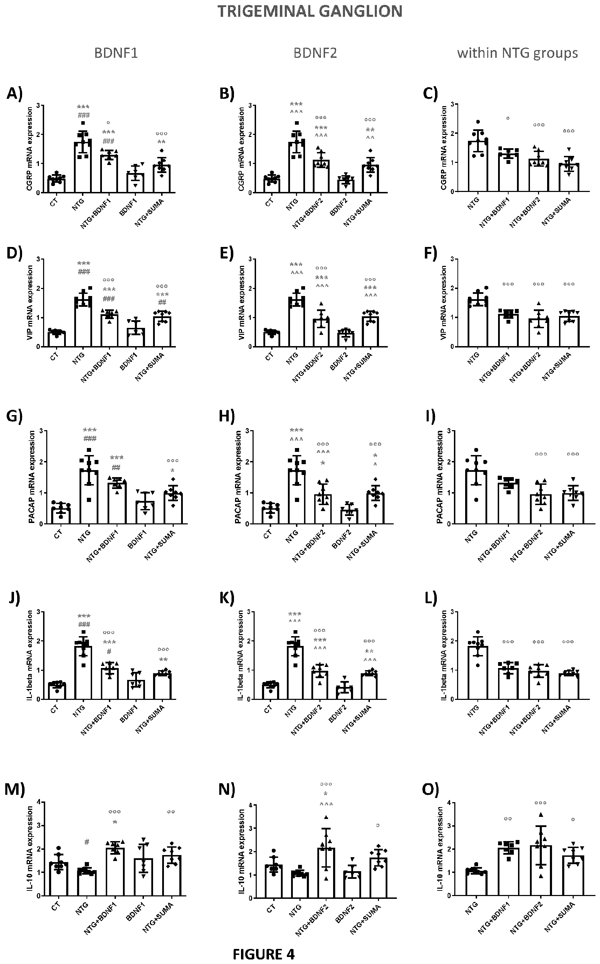

**Figure 4** shows the analysis in "CT", "NTG", "NTG+BDNF1", "BDNF1" and "NTG+SUMA" rat groups of the mRNA expression of CGRP (panel A), VIP (panel D), PACAP38 (panel G), IL-1beta (panel J), IL-10 (panel M) in the trigeminal ganglion; the analysis in "CT", "NTG", "NTG+BDNF2", "BDNF2" and "NTG+SUMA" rat groups of the mRNA expression of CGRP (panel B), VIP (panel E), PACAP38 (panel H), IL-1beta (panel K), IL-10 (panel N) in the trigeminal ganglion; the analysis in "NTG", "NTG+BDNF1", "NTG+BDNF2", and "NTG+SUMA" rat groups of the mRNA expression of CGRP (panel C), VIP (panel F), PACAP38 (panel I), IL-1beta (panel L), IL-10 (panel O) in the trigeminal ganglion. Data were normally distributed following Shapiro-Wilk test, thus the one-way ANOVA followed by post hoc Tukey's multiple comparison test was applied; *p<0.05, **p<0.01 and ***p<0.001 vs CT; °p<0.05, °°p<0.01 and °°°p<0.001 vs NTG; #p<0.05, ##p<0.01 and ###p<0.001 vs BDNF1 ; ^p<0.05, ^^p<0.01 and ^^^p<0.001 vs BDNF2.

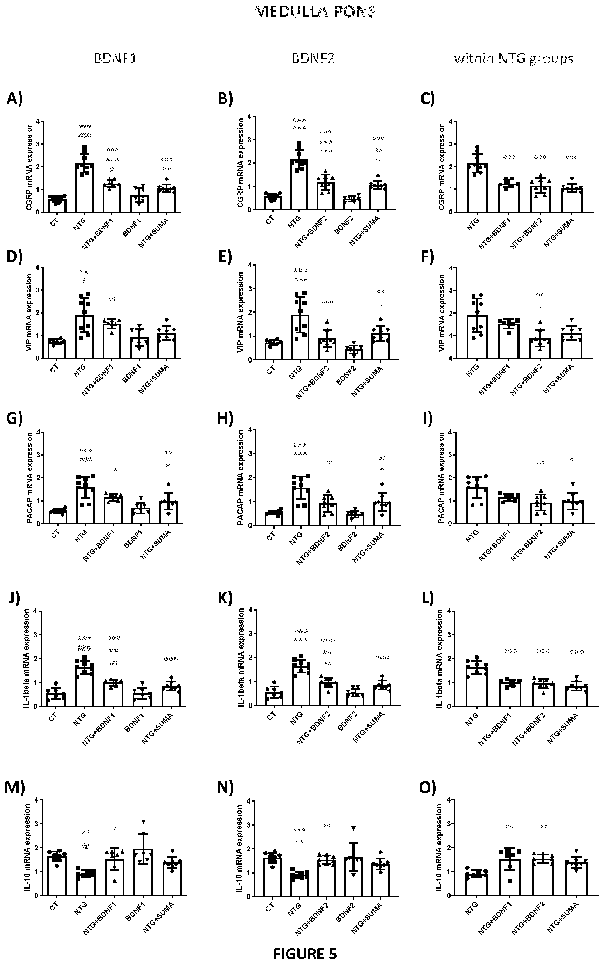

**Figure 5** shows the analysis in "CT", "NTG", "NTG+BDNF1", "BDNF1" and "NTG+SUMA" rat groups of the mRNA expression of CGRP (panel A), VIP (panel D), PACAP38 (panel G), IL-1beta (panel J), IL-10 (panel M) in the medulla-pons area; the analysis in "CT", "NTG", "NTG+BDNF2", "BDNF2" and "NTG+SUMA" rat groups of the mRNA expression of CGRP (panel B), VIP (panel E), PACAP38 (panel H), IL-1beta (panel K), IL-10 (panel N) in the medulla-pons area; the analysis in "NTG", "NTG+BDNF1", "NTG+BDNF2", and "NTG+SUMA" rat groups of the mRNA expression of CGRP (panel C), VIP (panel F), PACAP38 (panel I), IL-1beta (panel L), IL-10 (panel O) in the medulla-pons area. Data in A,B,C,E,G,H,I,J,K,L were normally distributed following Shapiro-Wilk test, thus the one-way ANOVA followed by post hoc Tukey's multiple comparison test was applied. Data in D,F,M,N,O were not normally distributed following Shapiro-Wilk test, thus the Kruskal-Wallis's test followed by Dunn's post-hoc test was applied; *p<0.05, **p<0.01 and ***p<0.001 vs CT; °p<0.05, °°p<0.01 and °°°p<0.001 vs NTG; #p<0.05, ##p<0.01 and ###p<0.001 vs BDNF1; ^p<0.05, ^^p<0.01 and ^^^p<0.001 vs BDNF2; +p<0.05 vs NTG+BDNF1.

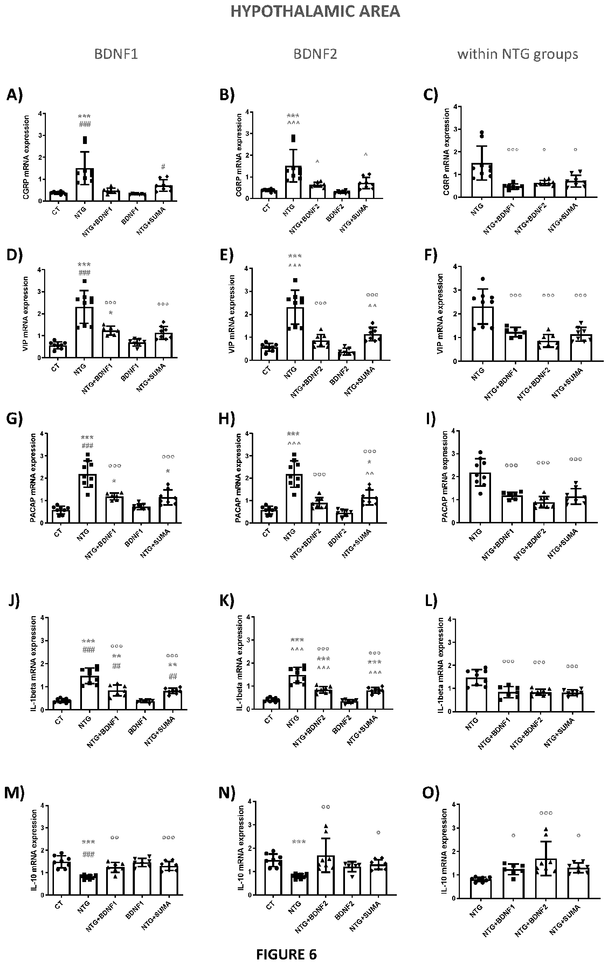

**Figure 6** shows the analysis in "CT", "NTG", "NTG+BDNF1", "BDNF1" and "NTG+SUMA" rat groups of the mRNA expression of CGRP (panel A), VIP (panel D), PACAP38 (panel G), IL-1beta (panel J), IL-10 (panel M) in the hypothalamic area; the analysis in "CT", "NTG", "NTG+BDNF2", "BDNF2" and "NTG+SUMA" rat groups of the mRNA expression of CGRP (panel B), VIP (panel E), PACAP38 (panel H), IL-1beta (panel K), IL-10 (panel N) in the hypothalamic area; the analysis in "NTG", "NTG+BDNF1", "NTG+BDNF2", and "NTG+SUMA" rat groups of the mRNA expression of CGRP (panel C), VIP (panel F), PACAP38 (panel I), IL-1beta (panel L), IL-10 (panel O) in the hypothalamic area. Data in D,E,F,G,H,I,J,K,L,M were normally distributed following Shapiro-Wilk test, thus the one-way ANOVA followed by post hoc Tukey's multiple comparison test was applied. Data in A,B,C,N,O were not normally distributed following Shapiro-Wilk test, thus the Kruskal-Wallis's test followed by Dunn's post-hoc test was applied; *p<0.05, **p<0.01 and ***p<0.001 vs CT; °p<0.05, °°p<0.01 and °°°p<0.001 vs NTG; #p<0.05, ##p<0.01 and ###p<0.001 vs BDNF1; ^p<0.05, ^^p<0.01 and ^^^p<0.001 vs BDNF2.

### DETAILED DESCRIPTION OF THE INVENTION

A first object of the invention is brain derived nerve factor (BDNF) for use in the acute or preventive treatment of cluster headache or migraine attacks in a subject, wherein said BDNF is administered intranasally to said subject.

The expression "preventive treatment", as used herein, means a treatment that, taken at regular intervals, lower CH or migraine attack frequency, duration and intensity, consequently minimizing the need for acute treatment.

The expression "acute treatment", as used herein, means a treatment that, taken at regular intervals, interrupts or aborts single attacks of migraine or CH.

Preferably, said subject is a human subject.

Preferably said BDNF is human BDNF, more preferably it is recombinant human BDNF (rhBDNF).

Preferably, the total amount of BDNF per each administration is between 50 µg and 1 mg, more preferably between 200 µg and 800 µg, even more preferably between 250 µg and 650 µg, divided in equal doses in each nostril.

Preferably, the BDNF for use according to the invention is administered from one to three times a day for a period of treatment of at least 7 days, preferably at least 60 days, more preferably at least 90 days.

The administration schedule may vary depending from the patient and it is selected by the skilled person based on the characteristics of the subject to be treated, the severity of the disease and the tests carried out during the treatment.

The evaluation of the above factors is within the knowledge and expertise of the skilled person.

A further object of the present invention relates to a pharmaceutical composition comprising BDNF, for use in the acute or preventive treatment of cluster headache or migraine attacks in a subject, wherein said composition is administered intranasally to said subject.

Preferably, the pharmaceutical composition according to the present invention comprises an effective amount of BDNF and at least one pharmaceutically acceptable excipient, preferably selected from solvents, thickening agents, mucoadhesive agents, buffers, antioxidants, preservatives, and penetration enhancers.

Preferably, the pharmaceutical composition according to the present invention is a liquid intranasal pharmaceutical composition.

Preferably, said BDNF is human BDNF, more preferably it is recombinant human BDNF (rhBDNF). Preferably, the concentration of BDNF in the pharmaceutical composition according to the invention is between 300 µg/ml and 1.5 mg/ml, more preferably between 500 µg/ml and 1.3 mg/ml.

Preferably, said solvent is water.

Preferably, said buffer is phosphate buffer.

A particularly preferred liquid intranasal pharmaceutical composition according to the invention comprises, preferably consists of, BDNF, phosphate buffer and water.

The pharmaceutical composition according to the invention may be suitably formulated using appropriate methods known in the art or by the method disclosed in Remington's Pharmaceutical Science (recent edition), Mack Publishing Company, Easton Pa.

In a further aspect, the present invention relates to a method for the acute or preventive treatment of cluster headache or migraine attacks in a subject comprising intranasally administering to the subject BDNF, as described above, in a therapeutically effective amount.

Preferably, said BDNF used in the method of the invention is in form of a pharmaceutical composition, as above defined.

The invention will be further described in the following examples, which do not limit the scope of the invention defined in the claims.

### EXPERIMENTAL SECTION

The inventors evaluated the effects of intranasal rhBDNF administration in an experimental animal model of primary headache associated with activation of the trigeminovascular system based on nitroglycerin (NTG) administration, which is representative of cluster headache and migraine.

Nitroglycerin administration is a well-known animal and human model of migraine, since it induces spontaneous-like migraine attacks in patients suffering from migraine (Demartini C. et al., Nitroglycerin as a comparative experimental model of migraine pain: From animal to human and back. Prog Neurobiol. 2019 Jun;177:15-32).

NTG is also known to induce CH-like attacks in CH patients during the active phases of disease (Sances G. et al., Reliability of the nitroglycerin provocative test in the diagnosis of neurovascular headaches. Cephalalgia. 2004 Feb;24(2):110-9).

This observation lends strength to the validity of NTG-induced attacks in migraine and CH subjects as a relevant model to investigate the neurobiological mechanisms involved in these primary headaches.

### Example 1

Male Sprague-Dawley rats weighing 200-270g (Charles River Laboratories Italia s.r.l, Calco, Lecco) were housed 2 per cage under controlled illumination (12 h light/dark cycle; light on 6:00 A.M.) and standard environmental conditions (ambient temperature 20-22°C, humidity 55-60%) for 1 week before starting of the experiments. Rats chow and tap water were available ad libitum. Animal care was carried out according to Italian (D.L. 116/92) and European Commission (O.J. of E.C. L358/1 18/12/86) regulations on the protection of labor tory animals. All efforts were made to reduce both animal numbers and suffering during the experiments.

The animals were randomly assigned to the following experimental groups of treatment, each comprising 9 animals, according to the experimental design reported in Figure 1:
1) NTG: treated with NTG 5mg/kg, i.p (5 total i.p. injections delivered every other day) and BDNF vehicle (4 total intranasal injections delivered on the days free from NTG injection);
2) CT: treated with NTG Vehicle (5 total i.p. injections delivered every other day) and BDNF vehicle (4 total intranasal injections delivered on the days free from NTG injection);
3) NTG+BDNF 1: treated with NTG 5mg/kg, i.p (5 total i.p. injections delivered every other day) and rhBDNF at high dose (80 µg/kg) (4 total intranasal injections delivered on the days free from NTG injection);
4) NTG+BDNF 2: treated with NTG 5mg/kg, i.p (5 total i.p. injections delivered every other day) and rhBDNF at low dose (40µg/kg) (4 total intranasal injections delivered on the days free from NTG injection);
5) BDNF 1: treated with NTG Vehicle (5 total i.p. injections delivered every other day) and rhBDNF at high dose (80 µg/kg) (4 total intranasal injections delivered on the days free from NTG vehicle injection);
6) BDNF 2: treated with NTG Vehicle (5 total i.p. injections delivered every other day) and rhBDNF at low dose (40µg/kg) (4 total intranasal injections delivered on the days free from NTG vehicle injection);
7) NTG+SUMA: treated with NTG 5mg/kg, i.p (5 total i.p. injections delivered every other day) and sumatriptan (5 mg/kg, i.p.; Imigran, 6 mg/0,5 ml, single injection delivered 1 hour after the last NTG injection).

In details, the animals of the above experimental groups were intraperitoneally (i.p.) injected with 5 mg/kg nitroglycerin (NTG) (Bioindustria L.I.M., Novi Ligure, Italy) (groups 1, 3 , 4 and 7) or its vehicle (NTG vehicle) (groups 2, 5 and 6), every other day over a 9-day period, for a total of 5 injections. The final volume of NTG or its vehicle injected in the animals was 1,5 mL/kg.

The NTG solution was prepared from a stock solution of 5.0 mg/1.5 mL of nitroglycerin dissolved in 27% alcohol and 73% propylene glycol; the thus-obtained NTG solution was diluted in saline (0.9% NaCl) to reach a final concentration of alcohol of about 6% and propylene glycol of 16%. The NTG vehicle solution was a 0.9% NaCl saline solution containing about 6% alcohol and 16% propylene glycol.

On the days free from NTG injection rhBDNF (stock solution 2,43mg/ml) or its vehicle (BDNF vehicle, 25 mM sodium dihydrogen phosphate dihydrate, 100 mM sodium chloride, pH 7.0) were intranasally administered for a total of 4 administrations (Figure 1) at final dosing of 40µg/kg (groups 4 and 6; volume ranging 3.54-4.85 µl, depending on rat weights, in a single nostril) or 80µg/kg (groups 3 and 5; volume ranging 6,72-9,6 µl, depending on rat weights, divided between nostrils) using the following procedure: after light anesthesia with 4% isoflurane in 5 % of oxygen (at a rate of 1.5 L/min) for 4-5 min for each rat), rats were placed in a supine position; rhBDNF, dissolved in phosphate buffer (25 mM sodium dihydrogen phosphate dihydrate, 100 mM sodium chloride, pH 7.0), was intranasally delivered as described below.

Using a dominant hand, a micropipette P10 was leaded with a predetermined quantity of compound. The tip of the filled pipette was placed near the rat's left nostril at 45-degree angle. The droplet was placed close enough to the nostril so that the rat can inhale the droplet. After 2 minutes from the inhalation from the left nostril, the same procedure was carried out to form another small droplet for the rat to inhale the compound through the other nostril.

Rats of the vehicle group received an equal amount 6,72-9,6 µl, depending on rat weights, (divided between nostrils) of BDNF vehicle (25 mM sodium dihydrogen phosphate dihydrate, 100 mM sodium chloride, pH 7.0) following the same procedure.

In group 7, the effect of sumatriptan (SUMA) was evaluated as a positive control for the putative effect of rhBDNF.

At day 9, two hours after the last administration of NTG or vehicle, animals were subjected to the orofacial formalin test, as described below, and subsequently they were sacrificed with a lethal dose of anesthetic (sodium thio-pental, 150mg/kg, i.p.) followed by decapitation, to evaluate neuropeptides (CGRP, PACAP38, VIP) and cytokines (IL-1β, IL-10) gene expression (in particular, mRNA expression) in medulla-pons region, hypothalamic area and trigeminal ganglia (by RT-PCR), as well as the concentrations of said neuropeptides in serum (by ELISA).

### Statistical Analysis

An a priori power analysis was conducted on the basis of previous studies (Greco R., et al., Effects of kynurenic acid analogue 1 (KYNA-A1) in nitroglycerin-induced hyperalgesia: Targets and antimigraine mechanisms. Cephalalgia. 2017 Nov;37(13):1272-1284; Greco R. et al., Characterization of the peripheral FAAH inhibitor,URB937, in animal models of acute and chronic migraine. Neurobiol Dis. 2021 Jan;147:105157; Greco R. et al., FAAH inhibition as a treatment for migraine: A pre-clinical study. Neurobiol Dis. 2020 Feb;134:104624.;Tomić MA., et al., The effects of levetiracetam, sumatriptan, and caffeine in a rat model of trigeminal pain: interactions in 2-component combinations. Anesth Analg. 2015 Jun;120(6):1385-93.) to calculate the minimal sample size needed to obtain a statistical power of 0.90 at an alpha level of 0.05 (GPower 3.1), thus estimating 6-9 rats per experimental group. Data were tested for normality utilizing the Kolmogorov-Smirnov test or Shapiro-Wilk normality test. Depending on data distribution a parametric (one-way ANOVA followed by post hoc Tukey's multiple comparisons test) or non-parametric (Kruskal-Wallis's test, followed by Dunn's post-hoc test tests for multiple comparisons) test was applied. A probability level of less than 5% was considered significant. Statistical significance was set at p < 0.05. All statistical analyses were performed using GraphPad Prism software (version 8). Data were expressed as mean ± standard deviation (SD).

### - Orofacial formalin test

Each rat was moved to the orofacial formalin test room, and after 5 min the animal received the subcutaneous injection of formalin (1.5%, 50 µL), an aqueous solution of 37% formaldehyde, performed into the right upper lip. Immediately after formalin injection, each rat was positioned into the observation box (a 30 × 30 × 30-cm glass chamber with mirrored sides) and its nocifensive behavior recorded for a 45-min period with a camera located at a distance of 50 cm from the box (Greco R. et al., Characterization of the peripheral FAAH inhibitor,URB937, in animal models of acute and chronic migraine. Neurobiol Dis. 2021 Jan;147:105157).

Face rubbing was measured (off-line) by counting the seconds the animal spent grooming the injected area with the ipsilateral forepaw or hind paw, 0-3 min (Phase I, reflecting acute pain) or 12-45 min (Phase II reflecting hyperalgesic pain) after formalin injection. The observation time was divided into 15 blocks of 3 min each.

Unexpectedly, the results reported in Figures 2A-F showed that even the lowest tested rhBDNF dose (NTG+BDNF2) was significantly effective in reducing NTG-induced pain sensitivity (hyperalgesia) (NTG); surprisingly, NTG+BDNF1, NTG+BDNF2 rat groups showed a very reduced NTG-induced pain sensitivity comparable to the rats treated with sumatriptan (NTG+SUMA).

The relevance of these findings is substantial, since intranasally-administered BDNF surprisingly exerts effects similar to sumatriptan, which is a synthetic drug with side adverse effects that impede a chronic administration for the acute or preventive treatment of migraine and CH attacks recurrence.

### - Serum concentration of VIP, PACAP38 and CGRP biomarkers

To assess if rhBDNF may have an effect also on the upregulation of the expression of vasoactive intestinal peptide (VIP), pituitary adenylate cyclase-activating polypeptide-38 (PACAP38) and calcitonin gene related peptide (CGRP) biomarkers, and on the increase of pro-inflammatory cytokines such as IL1β and decrease of anti-inflammatory cytokines such as IL-10, which have been widely reported in the serum of patients affected by migraine or CH at the end of the orofacial formalin test, the above-mentioned rat groups were sacrificed by decapitation as disclosed above, and, immediately after, the blood samples were collected in a clot activator with gel separator serum tubes and centrifuged for 15 min at 1000×g at 2-8 °C. Serum neuropeptides levels were measured using commercial ELISA kits (α-CGRP: Elabsciences, Houston, TX, USA; PACAP38 and VIP, Neo Biothech, Nanterre, France).

The results reported in Figures 3A-I showed that serum levels of VIP, PACAP38 and CGRP, which are notably involved in the activation of the trigeminal system in patients affected by migraine and cluster headache, were unexpectedly all dampened to baseline by both the tested concentrations of the intranasally administered rhBDNF (NTG+BDNF1 , NTG+BDNF2), thus reducing the serum levels of these biomarkers substantially to the levels of sumatriptan-treated rats (NTG+SUMA).

These serum data demonstrate that intranasally-administered rhBDNF is able to modulate the unbalanced levels of both vasoactive and nociceptive neuropeptides related to neurogenic inflammation in the pathogenesis of migraine and CH.

### - Gene expression of VIP, PACAP38 and CGRP biomarkers in medulla-pons area, hypothalamus and trigeminal ganglion.

Since the brain areas specifically involved in the development of cephalic pain bouts are the medulla-pons area, hypothalamus and trigeminal ganglion, these areas ipsilateral to the formalin injection were dissected out when animals were sacrificed as disclosed above, rinsed in sterile 0.9% NaCl cold solution, placed in cryogenic tubes and kept at -80 °C until rt-PCR processing for CGRP, PACAP38, VIP, interleukin-1beta (IL-1beta) and interleukin-10 (IL-10) gene expression (i.e., mRNA expression).

Following total RNA extraction (carried out by Aurum Fatty/Fibrous Tissue RNA Kit, 7326830, Bio-Rad), all RNA samples had absorbance ratios (260/280 nm) that varied from 1.9 to 2.0, indicating no significant protein contamination, including that from blood origin.

For quantitative real-time reverse-transcriptase PCR (qPCR), the 20µl reaction mixture (SsoFast EvaGreen Supermix , Bio-Rad) comprising 1.2 µl primers according to Table 1 (0.6 µl forward and 0.6 µl reverse primer), 1 µl cDNA (obtained by iScript cDNA Synthesis Kit, 1708891, Bio-Rad) and 10 µl of Syber Green Master and nuclease-free water (variable) mix was used.

Glyceraldehyde 3-phosphate dehydrogenase (GAPDH), whose expression remained constant in all experimental groups, was used for normalization. All samples were assayed in duplicate and the ΔΔCt method was used to investigate the differences in gene expression levels (Greco et al., 2022). The above-mentioned primers had the sequences (Merck) reported in the below Table 1, and were obtained from the Primer3 input (https://primer3.ut.ee/).

**Table 1: List of primers used for PCR, and SEQ ID Nos thereof.**

| **Gene** | **Forward primer** | **Reverse primer** |
|---|---|---|
| GAPDH | SEQ ID NO:1 AACCTGCCAAGTATGATGAC | SEQ ID NO:2 GGAGTTGCTGTTGAAGTCA |
| CGRP | SEQ ID NO:3 CAGTCTCAGCTCCAAGTCATC | SEQ ID NO:4 TTCCAAGGTTGACCTCAAAG |
| VIP | SEQ ID NO:5 CAGGCATGCTGATGGAGTTT | SEQ ID NO:6 TGCTTTCTAAGGCGGGTGTA |
| PACAP38 | SEQ ID NO:7 CAAGACTTCTATGACTGGGAC | SEQ ID NO:8 CTTCGTTAAGGATTTCGTGG |
| IL-1beta | SEQ ID NO:9 CTTCCTTGTGCAAGTGTCTG | SEQ ID NO:10 CAGGTCATTCTCCTCACTGTC |
| IL-10 | SEQ ID NO:11 GCTCAGCACTGCTATGTTGC | SEQ ID NO:12 CAGTAGATGCCGGGTGGTTC |

Each PCR reaction was performed in triplicate with the following parameters: Polymerase Activation 95°C for 30 secs (Cicle 1); Denaturation 95°C for 15 secs, (Cicles: 40); Annealing 60°C for 30 secs (Cicles: 40); Extension 72°C for 20 secs, (Cicles: 40), followed by a melting curve analysis (LightCycler^{®} 480 Software release 1.5.1.62 SP3).

Unexpectedly, a marked decrease in gene expression of VIP, PACAP38, CGRP, and IL1β in rhBDNF-treated animals, accompanied by an increase in gene expression of IL10 in trigeminal ganglion (Figures 4A-O) medulla-pons area (Figures 5A-O), and hypothalamic area (Figures 6A-O), was observed in rhBDNF-treated animals (NTG+BDNF1, NTG+BDNF2).

Notably, in rhBDNF-treated animals (NTG+BDNF1 and NTG+BDNF2), the brain transcriptional changes of VIP, PACAP38, CGRP showed in Fig. 4-6 were associated with the decrease in PACAP38, VIP and CGRP serum levels showed in Fig. 3, showing that rhBDNF affects the expression of mediators potentially involved in migraine and CH.

Taken together, these data show that said intranasal administration of rhBDNF is a feasible and pleiotropic therapeutic approach for the acute or preventive treatment of migraine and cluster headache patients through a safe non-invasive route of delivery.

## Claims

1. Brain derived nerve factor (BDNF) for use in the acute or preventive treatment of cluster headache or migraine attacks in a subject, wherein said BDNF is administered intranasally to said subject.

2. BDNF for use as claimed in claim 1, wherein said BDNF is human BDNF, preferably it is recombinant human BDNF (rhBDNF).

3. BDNF for use as claimed in claim 1 or 2, wherein said BDNF is administered from one to three times a day for a period of treatment of at least 7 days, preferably at least 60 days, more preferably at least 90 days.

4. BDNF for use as claimed in claims 1 to 3, wherein the total amount of BDNF per each administration is between 50 µg and 1 mg, preferably between 200 µg and 800 µg, more preferably between 250 µg and 650 µg, divided in equal doses in each nostril.

5. A pharmaceutical composition comprising BDNF and at least one pharmaceutically acceptable excipient, for use in the acute or preventive treatment of cluster headache or migraine attacks in a subject, wherein said composition is administered intranasally to said subject.

6. The pharmaceutical composition for use as claimed in claim 5, wherein said BDNF is human BDNF, preferably it is recombinant human BDNF (rhBDNF).

7. The pharmaceutical composition for use as claimed in claim 5 or 6, said BDNF is contained in the composition at a concentration between 300 µg/ml and 1.5 mg/ml, preferably between 500 µg/ml and 1.3 mg/ml.

8. The pharmaceutical composition for use as claimed in claims 5 to 7, wherein said pharmaceutical composition is a liquid intranasal pharmaceutical composition.

9. The pharmaceutical composition for use as claimed in claims 5 to 8, comprising, preferably consisting of, BDNF, phosphate buffer and water.
